# FASCICULE DE BREVET EUROPEEN

(11) **EP 2 432 522 B1**
(45) Date de publication et mention de la délivrance du brevet: **06.05.2015**
(21) Numéro de dépôt: 10718118.2
(22) Date de dépôt: 22.04.2010
(51) Int. Cl.: A61M 5/00, B65D 1/22, B65D 1/34, B65D 25/10

(54) **BOÎTE POUR LE STOCKAGE, LA PROTECTION ET LE TRANSPORT DE CONTENANTS ; CONTAINER, MÉTHODE DE PALETTISATION, MÉTHODE DE DÉ-PALETTISATION ET UTILISATION ASSOCIÉS**
BOX FÜR LAGERUNG, SCHUTZ UND TRANSPORT VON BEHÄLTERN ; VERBUNDENE PALETTE, PALETTIERUNGSMETHODE, DEPALETTIERUNGSMETHODE UND NUTZUNG
BOX FOR STORING, PROTECTING, AND TRANSPORTING CONTAINERS ; PALLET, PALLETIZING METHOD, DE-PALLETIZING METHOD, AND USAGE ASSOCIATED

(30) Priorité: 22.04.2009 BE 200900249
(43) Date de publication de la demande: 28.03.2012
(73) Titulaire: FLEXIWAYS S.P.R.L., B-1348 Louvain-La-Neuve (BE)
(72) Inventeur: LEPOT, Eric, B-1348 Louvain-La-Neuve (BE)
(74) Mandataire: Pecher, Nicolas
(86) Numéro de dépôt international: PCT/EP2010/055399
(87) Numéro de publication internationale: WO 2010/122129

(56) Documents cités:
- EP-A- 1 088 566
- WO-A-99/45985
- WO-A1-2009/053434
- BE-A5- 1 017 811
- DE-A1-102007 028 009
- DE-U1- 29 814 026
- DE-U1- 29 910 076
- DE-U1-202007 005 651
- US-A- 4 548 824
- US-A- 4 944 730
- US-A- 6 012 595

## Description

### OBJET DE L'INVENTION

La présente invention concerne une boîte pour le stockage, le transport et la protection de contenants. L'invention concerne, plus particulièrement, une boîte pour le stockage, la protection et le transport de contenants suspendus en position verticale tels que par exemple des contenants de produits médicaux. L'invention concerne également un container pour le transport desdites boîtes, une méthode de palettisation et dé-palettisation automatique et une utilisation des boîtes.

### ARRIERE-PLAN TECHNOLOGIQUE

Actuellement, les contenants de produits médicaux, tels que des seringues en verre, sont conditionnés par les fabricants dans une forme appelée « nest » et livrés dans des conditions stériles. Livrer des seringues sous un format « nest », signifie livrer des seringues qui ont été emballées sur un plateau en plastique comprenant principalement une plaque de support, nommé, ci-après, plateau nest qui est formé d'une matrice d'ouvertures, chacune de ces ouvertures supportant une seringue. Ce plateau nest est typiquement supporté par une boîte en plastique nommé, ci-après, boîte nest qui protège les seringues (voir par exemple le produit **HYPAK^{®} SCF^{®},** Becton, Dickinson and Company of Franklin Lakes, New Jersey, USA). Ce conditionnement est, de plus, recouvert par un opercule, mis en sachet, et est alors prêt pour la stérilisation.

Quand les seringues sont livrées sous le format « nest » à une société pharmaceutique, elles doivent être manipulées pour être remplies une à une. Après avoir enlevé le sachet et l'opercule de chaque conditionnement, manuellement ou au moyen d'équipements automatisés, chaque plateau nest est retiré de sa boîte nest correspondante et les seringues sont alors remplies une à une, ligne par ligne ou par série de plusieurs pièces. Après l'étape de remplissage, il est nécessaire d'une part, d'étiqueter chaque seringue dans le but de définir le produit médical contenu et d'autre part, d'inspecter les seringues pour détecter toute contamination du produit ainsi que les fissures ou griffes éventuelles dans les seringues. D'autres opérations peuvent être requises comme par exemple l'ajout de protections d'aiguilles ou de bouchons. L'inspection est classiquement réalisée au moyen d'un système de capteurs optiques (ou d'une caméra) qui inspectent une par une les seringues. Cependant, en accord avec la pratique habituelle et en raison de limitations techniques (quantité de seringues, difficultés à étiqueter et/ou à inspecter les seringues placées dans la partie centrale du plateau nest...etc.), les seringues sont d'abord enlevées du plateau nest avant d'être étiquetées et/ou contrôlées. Ainsi, lorsque les seringues sont finalement étiquetées et /ou contrôlées, elles sont alors encore manipulées et stockées soit directement dans la boîte nest sans être stockées sur le plateau nest, soit dans un support différent nommé, ci-après, plateau peigne (voir le document US 6,012,595 décrit ci-après), ce dernier possède de multiples doigts allongés pour garder les seringues suspendues par leurs collerettes. Il doit être remarqué qu'après l'étiquetage et/ou l'inspection, les seringues ne sont plus réinsérées dans le plateau nest parce qu'il est plus efficient et facile de les stocker dans le plateau peigne qui est acheté séparément. Un conditionnement de type nest, tel que le produit **HYPAK^{®} SCF^{®},** n'est donc pas adapté pour une manipulation automatisée car il requiert des machines complexes. D'autre part, dans le cadre d'une manipulation semi-automatique (avec assistance humaine), il est difficile de dépasser les cadences de plus de 6000 seringues par heure. Cependant, si d'une part, les plateaux peignes sont préférables aux plateaux nests pour stocker les seringues de façon plus rapide et plus aisée, ils ne sont, d'autre part, pas adaptés au transport et au stockage des seringues. En effet, quand les plateaux peignes sont stockés les uns sur les autres en colonnes verticales, certaines parties des seringues risquent d'être endommagées ou brisées, en raison de leur contact direct avec le dessous du conditionnement posé au-dessus. Afin d'éviter ce problème, les plateaux peignes doivent être placés dans des boîtes spécifiques supplémentaires et munis de couvercles avec un rebord intérieur de blocage.

Des boîtes spécifiques dans lesquelles sont disposés les contenants médicaux sont connues dans l'art mais celles-ci ne sont pas adaptées, du fait de leur structure, à toutes les étapes automatisées de la chaîne de production. Ainsi, une boîte ayant une structure trop souple, pas assez rigide, empêchera par exemple la bonne efficacité d'une étape de palettisation ou de dé-palettisation puisque celle-ci seront difficilement manipulable par un système automatisé.

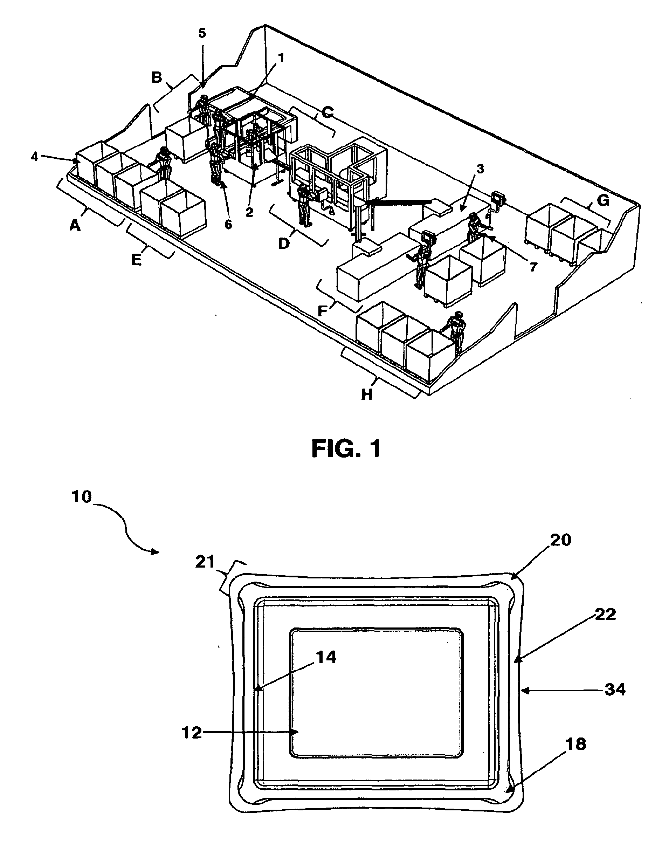

En effet, la FIG. 1 représente une vue schématique d'une ligne de production telle que connue actuellement. Celle-ci détaille les différentes étapes entreprises pour le contrôle ou l'inspection de seringues de vaccin. Le contrôle de la qualité de la seringue s'effectue à l'aide de caméra et de contrôleur optique. L'étape de la zone A correspond au déplacement d'une palette 4 comprenant des boîtes « nest » remplies de seringues. La palette est amenée dans une zone B par un opérateur 5 qui place les boîtes « nest » sur un convoyeur d'accumulation 1 chargé d'approvisionner le robot 2 de la zone C avec les boîtes. Le robot 2 de la zone C enlève les seringues de la boîte « nest » pour les envoyer vers la zone de contrôle ou d'inspection D par l'intermédiaire d'un nouveau convoyeur. Dans le même temps un opérateur 6 récupère les boîtes « nest » vidées pour les remettre manuellement dans une palette en zone E. Les seringues sont analysées par les caméras et/ou les contrôleurs optiques dans la zone D puis acheminées en zone F où lesdites seringues sont rangées sur des peignes puis dans des boîtes et surmontées d'un couvercle par l'intermédiaire des appareillages 3. Ces derniers sont alimentés en peignes, boîtes et couvercles par des opérateurs 7 qui acheminent manuellement les peignes vides, les boîtes vides et les couvercles de la zone G à la zone F. La dernière étape consiste à acheminer les boîtes remplies des seringues disposées sur les peignes et fermées par le couvercle de la zone F à H. Les boîtes utilisées dans les zones F et G et celles utilisées dans les zones A et B ne sont pas les mêmes du fait de la difficulté de manipulation des boîtes de type nest par des systèmes automatisés à haute cadence. Cette ligne de production, généralement soumise à des normes strictes de propreté et d'hygiène, (Grade A à D, norme GMP) est consommatrice d'espace, de consommables, d'opérateurs, et d'énergie du fait des problèmes liés au chevauchement des boîtes dans les palettes. Généralement, lors des changements de palettes, la ligne de production doit être arrêtée créant ainsi un problème d'intervalle d'arrêt. Les grades A et B correspondent à un nombre maximum de 3.500 particules de 0,5 µm et plus par m³ et 0 particules au-delà de 5 µm. Le grade C correspond à un nombre maximum de 350.000 particules de 0,5 µm et plus par m³ et 2.000 particules au-delà de 5 µm. Le grade D correspond à un nombre maximum de 3.500.000 particules de 0,5 µm et plus par m³ et 20.000 particules au-delà de 5 µm.

Les problèmes liés au chargement ou au déchargement d'une palette n'ont pas pu être résolus. Des systèmes comprenant une plaque entre chaque étage de la palette ont pu être envisagés mais cela nécessite en l'utilisation d'un dispositif de pince préhension complexe. Cette étape de palettisation ou de dé-palettisation est donc généralement effectuée manuellement soulevant ainsi toutes les questions liées à la qualité des produits suite à la manipulation manuelle : possibilité de contamination des contenants, risque de dommages liés à la manipulation...

Il est clair qu'il y a là un besoin d'une boîte capable de résoudre les limitations et les inconvénients des systèmes actuels et de permettre de procéder plus aisément et rapidement au stockage, transport et livraison de contenants médicaux. Il existe, en particulier, le besoin d'une boîte qui permette le support de seringues suspendues en position verticale de façon plus aisée et plus sécurisée, qui rende plus efficace et facile la manipulation de grandes quantités de seringues au cours des processus de stérilisation, de remplissage, d'étiquetage et/ou d'inspection, réalisée par des machines hautement automatisées dans les lignes de production.

L'art antérieur est également divulgué dans les documents WO 2009/053434 A1, DE 20 2007 005651 U1 et DE 299 10 076 U1.

### ETAT DE LA TECHNIQUE

On connaît par le document US 6,012,595 un système pour le stockage de seringues. Ce système de stockage comprend une boîte et un plateau fait d'un ensemble de doigts allongés reliés à une arrête commune. Ces doigts dessinent plusieurs canaux parallèles qui supportent les seringues par leurs collerettes. Cependant, ce système de stockage n'est pas capable de supporter les seringues quand le plateau est retiré de la boîte et tourné verticalement tête en bas. De plus, ce plateau requiert une boîte spécifique comportant un couvercle muni d'un rebord intérieur de blocage pour fixer ledit plateau car les canaux ne sont pas assez rigides et peuvent être aisément déformés, ce qui peut entraîner la chute des seringues et leur endommagement. En outre, ce système de stockage ne convient pas à une manipulation automatique à haute cadence, parce qu'il requiert l'utilisation d'une machine automatique complexe capable de manipuler cette boîte spécifique et ce plateau particulier. Enfin, ce système de stockage nécessite un type particulier de plateau pour un type donné de seringues (c'est-à-dire une seringue avec un diamètre donné ou une seringue avec un type de collerette donné), dans le but de protéger les seringues contre les chutes et les dommages. Ceci ayant comme conséquence que pour être compatible avec les quatre types de seringues les plus couramment utilisées, il est nécessaire de fabriquer quatre différents types de systèmes de stockage.

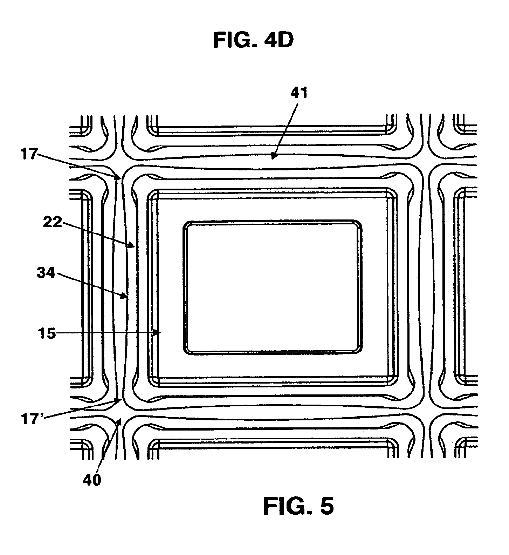

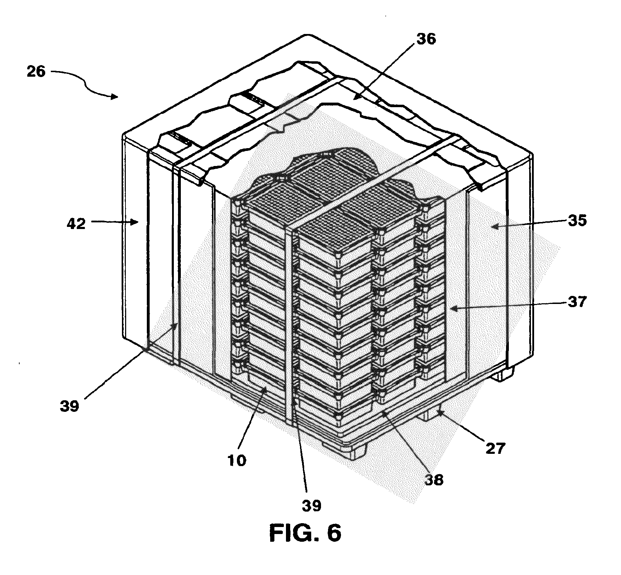

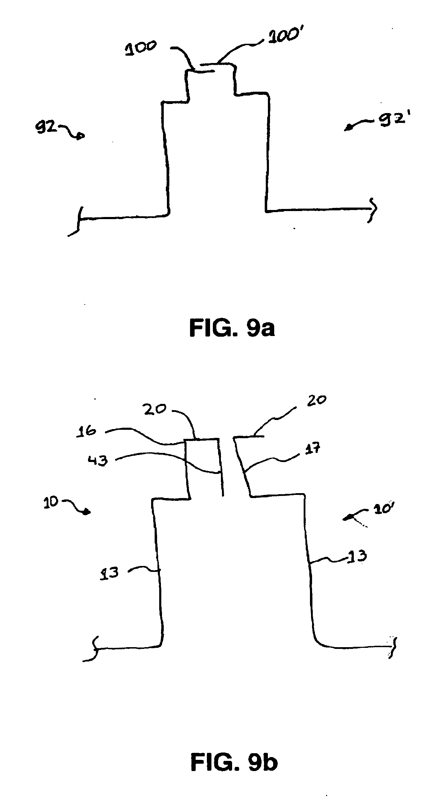

Ce même document US 6,012,595 décrit une boîte de stockage pour seringues (élément 50 de la Fig. 6(b) de ce document) configurée pour soutenir un plateau en forme de peigne et comportant des portions pour restreindre le mouvement dudit plateau. Il est également connu par WO 99/45985, (voir Fig. 5 de ce document) une boîte 92 pour le stockage de seringues. La boîte comprend un fond 96, des parois latérales inférieures 94 se raccordant audit fond, et un redan 98 raccordé auxdites parois latérales inférieures 94. Ladite boîte 92 comprend également des parois latérales supérieures raccordées audit redan 98 et à un rebord supérieur 100. Ce rebord 100 s'étend sur toute la périphérie de l'ouverture supérieure des parois latérales supérieures. Il permet le scellement d'une feuille 104 assurant la fermeture du conteneur. Cependant, comme représenté à la **Fig 9a****,** la présence de ce rebord 100 a pour conséquence qu'une boîte 92 placée sur une surface horizontale au voisinage d'une boîte identique 92' sur un plan horizontal présente un bord mince du rebord 100 à côté du bord mince du même rebord 100' de l'autre boîte, de sorte qu'une faible inégalité de hauteur des deux boîtes pourra conduire à un chevauchement des deux boîtes. L'épaisseur du rebord 100 ne permet pas d'éviter le chevauchement. Ces boîtes de stockage présentent donc des inconvénients de manipulation et de stockage puisque leur configuration ne permet pas d'éviter le chevauchement avec une autre boîte du même type. En outre, ces boîtes ne présentent pas de moyens facilitant leur manipulation automatisée à haute cadence indispensable dans le cadre visé.

La présente invention a pour but de fournir une boîte pour stocker, protéger et transporter des objets stérilisés ou à stériliser ne présentant pas tous les désavantages de l'art antérieur. En particulier, l'invention a pour but de proposer une boîte pour stocker, protéger et transporter de façon sécurisée une grande quantité de contenants médicaux et qui évite le chevauchement d'une boîte sur une autre lors de la manipulation, du stockage ou du transport, tout en permettant un rangement des boîtes les unes au-dessus des autres. Un autre but de la présente invention est de fournir une boîte permettant de soutenir les contenants en position suspendue verticale et inversée. Encore un autre but de la présente invention est de fournir une boîte qui puisse être aisément manipulée par des moyens automatisés dans le but d'enlever les contenants desdits dispositifs de façon rapide et sécurisée. Finalement, un autre but de la présente invention est de fournir une boîte compatible avec des seringues indépendamment de leur longueur, type de collerette et diamètre, par exemple acceptant indifféremment des seringues de 6,85 mm et de 8,15 mm de diamètre ou présentant des flasques de formes différentes.

De nos jours, les conditionnements des contenants de produits médicaux liquides sont typiquement fournis sous forme d'un bloc de 1200 X 1000 mm, format palette (format EuroPallet), en accord avec la norme UIC 435-2. Ce qui signifie que plusieurs conditionnements de contenants sont disposés l'un à côté de l'autre et l'un au-dessus de l'autre. Donc, un autre but de la présente invention est de fournir une boîte pour stocker, protéger et transporter des contenants médicaux et qui convienne à un rangement les uns à côté des autres et en colonne les uns au-dessus des autres et protégeant lesdits contenants de dégâts ou de contaminations. Un autre but de l'invention est de fournir un container pour transporter des boîtes permettant de soutenir les contenants en position suspendue verticale et inversée. Finalement, la présente invention a également pour but de fournir une méthode de palettisation et de dé-palettisation.

### RESUME DE L'INVENTION

Selon un premier aspect de l'invention, une boîte pour le stockage, le transport et la protection de contenants en position suspendue verticale est fournie. Ladite boite comprend une ouverture supérieure, un fond rectangulaire, des parois latérales inférieures se raccordant audit fond, un redan se raccordant auxdites parois latérales inférieures et formant un appui pour supporter un plateau, des parois latérales supérieures se raccordant par leur extrémité inférieure audit redan et par leur extrémité supérieure à un rebord supérieur s'étendant vers l'extérieur de la boîte. Suivant l'invention, une bordure est positionnée sur les coins supérieurs de la boîte et les parois latérales supérieures de la boîte comportent au moins une portion s'étendant vers l'extérieur de la boîte, de sorte que ladite boîte, lorsqu'elle est disposée côte à côte avec une seconde boîte, est en contact avec ladite seconde boîte par l'intermédiaire de ladite portion, empêchant ainsi le chevauchement de ladite boîte avec ladite seconde boîte.

Suivant un premier mode de réalisation préféré, ladite portion est une jupe raccordée au rebord supérieur.

Ladite jupe peut s'étendre sur toute la périphérie de la boîte.

Suivant second mode de réalisation préféré, ladite portion est raccordée au redan.

Ladite portion peut également s'étendre verticalement, sensiblement verticalement, obliquement ou obliquement inversée. Dans ce cas, le risque de chevauchement entre deux boîtes selon l'invention disposée l'une à côté de l'autre est minimum.

De préférence, ladite au moins une portion peut être située dans un coin de ladite boîte. Préférentiellement, ladite au moins une portion peut être située dans un coin supérieur de ladite boîte. Avantageusement, ladite boîte selon la présente invention peut contenir quatre parois latérales supérieures comportant chacune au moins une portion s'étendant vers l'extérieur. Ainsi, ladite boîte peut contenir au moins quatre portions s'étendant vers l'extérieur. Plus préférentiellement, lesdites au moins quatre portions peuvent être situées dans les coins supérieurs de ladite boîte.

De préférence, ladite boîte peut comprendre dans ses parois latérales supérieures au moins un évidement pour saisir et retirer aisément ledit plateau de ladite boîte. Avantageusement, ledit au moins un évidement peut être situé dans un coin de ladite boîte. De préférence, ledit au moins un évidement peut être situé dans un coin supérieur de ladite boîte. Ledit évidement peut contenir une ou plusieurs protubérances, un ou plusieurs renfoncements ou une ou plusieurs cavités.

Avantageusement, ladite boîte peut avoir une forme parallélépipédique.

Plus avantageusement, ladite boîte peut comporter des perforations dans lesdites parois latérales supérieures ou lesdites parois latérales inférieures pour faciliter la stérilisation de ladite boîte et de son contenu. La stérilisation de ladite boîte et de son contenu peut être effectuée au moyen d'un procédé de stérilisation à la vapeur, par l'ETO, ou par irradiation.

De préférence, ladite bordure peut être de forme concave, convexe, ondulée, plane ou être de toute autre forme susceptible de favoriser et d'améliorer sa manipulation par un dispositif automatique. De préférence, ladite bordure est de forme plane.

De préférence, ladite bordure et ledit rebord supérieur raccordé à une des parois latérales supérieures peuvent comporter au moins un point de jonction. Avantageusement, ladite bordure et ledit rebord supérieur peuvent être approximativement dans le même plan. De préférence, ladite bordure peut s'étendre vers l'intérieur de la boîte. Alternativement, ladite bordure peut s'étendre vers l'extérieur de la boîte et peut comporter une paroi verticale. Ladite paroi verticale peut également s'étendre le long du rebord supérieur.

Préférentiellement, ladite bordure et ledit rebord supérieur peuvent former une surface continue. Ladite bordure peut comporter sur certaines parties des saillies, des aspérités ou des renfoncements pour favoriser et améliorer sa manipulation par un dispositif automatique.

Selon un mode de réalisation préféré de l'invention, ledit rebord supérieur peut avoir un bord périphérique de forme concave ou convexe. De préférence, le bord périphérique peut être de forme concave. Selon l'invention, ledit bord périphérique est situé en deçà du plan vertical formé par deux desdites portions. Ainsi, le contact entre des boîtes selon l'invention disposée l'une à côté de l'autre ne peut pas se faire par le bord périphérique du rebord supérieur ; un espace est alors crée entre deux boîtes, évitant le chevauchement de celles-ci. Alternativement, lesdites portions s'étendent verticalement, sensiblement verticalement, obliquement ou obliquement inversée sur tout ou partie du rebord supérieur.

De préférence, ladite bordure et ledit rebord supérieur peuvent être surmontés par un opercule ou un couvercle pour favoriser l'empilage desdites boîtes les unes sur les autres et limiter les échanges entre l'intérieur et l'extérieur desdites boîtes. L'opercule peut être en fibres de polyéthylène haute densité, tel que du Tyvek®. L'opercule peut être collé ou soudé à ladite bordure et audit rebord supérieur.

De préférence, ladite boîte peut contenir un plateau pour le support de contenants. Selon un mode de réalisation de l'invention, ledit plateau peut être un plateau comprenant une pluralité de rails disposés parallèlement les uns aux autres sur une face dudit plateau, des lèvres de support étant agencées le long desdits rails, une lèvre et un rail formant ainsi avec une lèvre d'un rail adjacent une ouverture, une paire de lèvres adjacentes étant aptes à soutenir et stocker des contenants par leurs flasques, et en ce que ledit plateau comprend une paroi supérieure perpendiculaire à ladite pluralité de rails et que lesdits rails et lesdites lèvres sont en substance libre d'aspérités, permettant ainsi aux contenants de glisser le long des rails. Ladite pluralité de rails peut être fixée de façon rigide à ladite paroi supérieure de sorte que lesdites ouvertures ne se déforment pas, empêchant ainsi lesdits contenants de tomber. Le plateau peut ainsi comporter des rails secondaires pour empêcher les contenants de se déplacer latéralement par rapport auxdites ouvertures. La paroi supérieure dudit plateau peut comporter une pluralité de passages pratiqués en regard desdites ouvertures pour permettre l'accès à une face supérieure desdits contenants. Ledit plateau peut également comporter une languette de maintien configuré pour recevoir une pluralité de contenants par enclipsage, et pour positionner lesdits objets en regard desdits passages. De plus, ce type de plateau permet d'éviter que lesdits contenants sortent dudit plateau lorsque ce dernier pivote autour d'un axe horizontal. Ce plateau présente l'avantage de pouvoir être extrait de ladite boîte par un système de ventouse.

Selon un autre mode de réalisation, ledit plateau peut être un plateau connu dans l'art tel qu'un plateau nest ou un plateau en forme de peigne.

Selon un mode de réalisation préféré, ledit plateau peut être surmonté d'au moins une feuille de protection scellée ou non. La feuille de protection peut être en fibres de polyéthylène haute densité.

Selon un second aspect de l'invention, un container pour le transport de boîtes selon le premier aspect de l'invention est fourni. Ledit container comprend une palette sur laquelle est disposée une pluralité de boîtes selon l'invention et contenant optionnellement un moyen de contrôle de la qualité dudit container. Ladite palette peut être une palette EuroPallet aux dimensions normalisées de 800 mm de largeur par 1200 mm de longueur. De préférence, ladite palette peut être une palette plastique.

Selon un mode de réalisation préféré, ledit container peut comprendre également des parois latérales verticales et un couvercle rigide. Les parois latérales verticales sont également connues sous le terme « ceintures rigides de supportage ». Lesdites parois latérales verticales peuvent être en polyéthylène et peuvent servir à maintenir ladite pluralité de boîte sur la palette pendant le transport ou le convoyage du container. Le couvercle rigide peut servir à empiler lesdits containers les uns au dessus des autres. Ledit container peut également comprendre une housse, une ou plusieurs sangles ou une platine de localisation.

Selon un mode de réalisation préféré de l'invention, ladite pluralité de boîtes peut être contenue dans ou entourée par un élément de protection. Ledit élément de protection peut être un sac ou un emballage. De préférence, ledit élément de protection est configuré pour maintenir ladite pluralité de boîtes à une pression inférieure à la pression atmosphérique. La pression atmosphérique considérée dans la présente invention est de 1013 hPa. Plus préférentiellement, ledit élément de protection est configuré pour maintenir ladite pluralité de boîtes en dépression. Le terme « dépression » tel qu'utilisé dans la présente invention se réfère à une pression inférieure à 500 hPa. Ladite pluralité de boîtes est alors maintenue en un bloc rigide.

De préférence, ledit moyen de contrôle de la qualité dudit container peut être ledit élément de protection entourant ladite pluralité de boîtes. Alternativement, ledit moyen de contrôle de la qualité dudit container peut être un ou plusieurs scellés, un ou plusieurs cerclages, une ou plusieurs housses, une ou plusieurs sangles, un couvercle ou une ou plusieurs parois entourant ledit container.

Selon un autre aspect, l'invention se rapporte à une méthode de palettisation automatique caractérisée en ce qu'elle comprend les étapes suivantes de :
- mise à disposition d'une pluralité de boîtes selon l'invention et une palette,
- disposer, à l'aide d'un dispositif automatisé, sur ladite palette ladite pluralité de boîtes selon l'invention, côte à côte ou l'une sur l'autre, tel que les portions des parois latérales supérieures desdites boîtes s'appuient les unes contre les autres les empêchant de se chevaucher,
- fournir un container selon la présente invention.

Avantageusement, ledit dispositif automatisé manipule lesdites boîtes par l'intermédiaire de leurs parois latérales inférieures, leurs parois latérales supérieures, leurs évidements ou par lesdites bordures positionnées sur les coins supérieurs de ladite boîte.

Selon un mode de réalisation préféré de l'invention, ladite méthode de palettisation automatique comprend également l'étape d'emballer lesdites boîtes disposées sur la palette d'un élément de protection. De préférence, ledit élément de protection peut être un sac ou un emballage. De préférence, ledit élément de protection est configuré pour maintenir ladite pluralité de boîtes à une pression inférieure à la pression atmosphérique. Plus préférentiellement, ledit élément de protection est configuré pour maintenir ladite pluralité de boîtes en dépression. Lorsque ledit élément de protection est configuré pour maintenir ladite pluralité de boîtes en dépression, celui-ci joue le rôle de moyen de contrôle de la qualité dudit container. En effet, si lors de l'utilisation dudit container, ladite pluralité de boîtes n'est plus en dépression, cela implique que l'élément de protection entourant lesdites boîtes n'est plus étanche et donc que lesdites ont pu être en contact avec un élément extérieur pouvant altérer sa qualité, sa stérilité. Si plusieurs moyens de contrôle de la qualité ont été utilisés, la vérification peut se faire à différentes étapes de la procédure. Le maintien de condition stérile peut, par exemple, être important dans le domaine pharmaceutique ou médical.

Selon un autre mode de réalisation préféré de l'invention, ladite méthode de palettisation automatique comprend également l'étape d'ajout de parois latérales verticales et d'un couvercle rigide. Lesdites parois latérales verticales peuvent être en polyéthylène et peuvent servir à maintenir ladite pluralité de boîte sur la palette pendant le transport, le convoyage du container. Le couvercle rigide peut servir à empiler lesdites boîtes les unes au dessus des autres.

Selon un autre aspect, l'invention se rapporte à une méthode de dé-palettisation automatique caractérisée en ce qu'elle comprend les étapes suivantes de :
a) mise à disposition d'un container selon l'invention comprenant une palette sur laquelle est disposée une pluralité de boîtes selon l'invention,
b) retirer ladite pluralité de boîtes de ladite palette par l'intermédiaire d'un système automatisé manipulant lesdites boîtes par l'intermédiaire de leurs parois latérales inférieures, leurs parois latérales supérieures 16, leurs évidements ou par lesdites bordures positionnées sur les coins supérieurs de ladite boîte.

Selon un mode de réalisation préféré de l'invention, ladite méthode de dé-palettisation automatique comprend également une étape antérieure à l'étape b) de retrait dudit élément de protection entourant ladite pluralité de boîtes.

Selon un mode de réalisation préféré de l'invention, ladite méthode de dé-palettisation automatique comprend également une étape antérieure à l'étape b) de retrait des parois latérales verticales et du couvercle rigide entourant ladite pluralité de boîtes.

Selon un autre aspect de l'invention, la boîte, selon le premier aspect de l'invention, est utilisée pour le stockage, le transport et la protection de contenants suspendus dans une position verticale. De préférence, lesdits contenants suspendus sont des contenants de produits médicaux. Plus particulièrement, les contenants de produits médicaux peuvent être des seringues, des flacons ou de tout autre objet muni de flasques ou de collerettes.

D'autres aspects et avantages de formes d'exécution de l'invention seront discutés avec référence aux figures et à la description détaillée des formes d'exécution préférées.

### Brève description des figures

La Fig. 1 représente une vue schématique d'une ligne de production connue pour le contrôle de contenants médicaux.
La Fig. 2 représente une vue du dessus d'une boîte selon l'invention.
LA Fig. 3 représente une vue latérale en coupe d'une boîte selon l'invention.
La Fig. 4A représente une vue en perspective d'une coupe d'une boîte selon l'invention.
Les Fig. 4B et 4C représentent une vue en coupe d'un coin de la boîte selon l'invention.
La Fig. 5 représente une vue de dessus d'un ensemble de boîtes selon l'invention.
La Fig. 6 représente une vue en perspective d'un container suivant l'invention contenant une pluralité de boîtes selon l'invention.
La Fig. 7 représente une vue latérale en coupe d'une boîte suivant l'invention contenant un plateau.
La Fig. 8 représente une vue schématique d'une ligne de production pour le contrôle de contenants médicaux et utilisant des containers et des boîtes selon l'invention.
La Fig. 9a représente partiellement et en coupe deux boîtes suivant l'art antérieur placées côte à côte, et susceptibles de se chevaucher. La Fig. 9b représente partiellement et en coupe deux boîtes suivant l'invention placées côte à côte.

### Description détaillée de l'invention

Selon un premier aspect de l'invention, une boîte pour le stockage, le transport et la protection de contenants est fournie. Ladite boîte comprend une ouverture supérieure, un fond rectangulaire, des parois latérales inférieures se raccordant audit fond, et un redan se raccordant auxdites parois latérales inférieures et formant un appui pour supporter un plateau, caractérisée en ce que ladite boîte comprend des parois latérales supérieures raccordées audit redan et chacune comportant au moins une portion s'étendant vers l'extérieur de la boîte, chacune desdites parois latérales supérieures étant également raccordées à un rebord supérieur s'étendant vers l'extérieur de la boîte, et en ce qu'une bordure est positionnée sur les coins supérieurs de ladite boîte. La boîte selon l'invention permet d'éviter et d'empêcher tout chevauchement avec une autre boîte du même type, facilitant ainsi leur manipulation à haute cadence. En effet, le contact entre deux boîtes selon l'invention s'effectue de préférence par lesdites portions des parois latérales supérieures de sorte que même si un défaut d'alignement dans le plan vertical existe entre lesdites deux boîtes, aucun chevauchement n'est constaté.

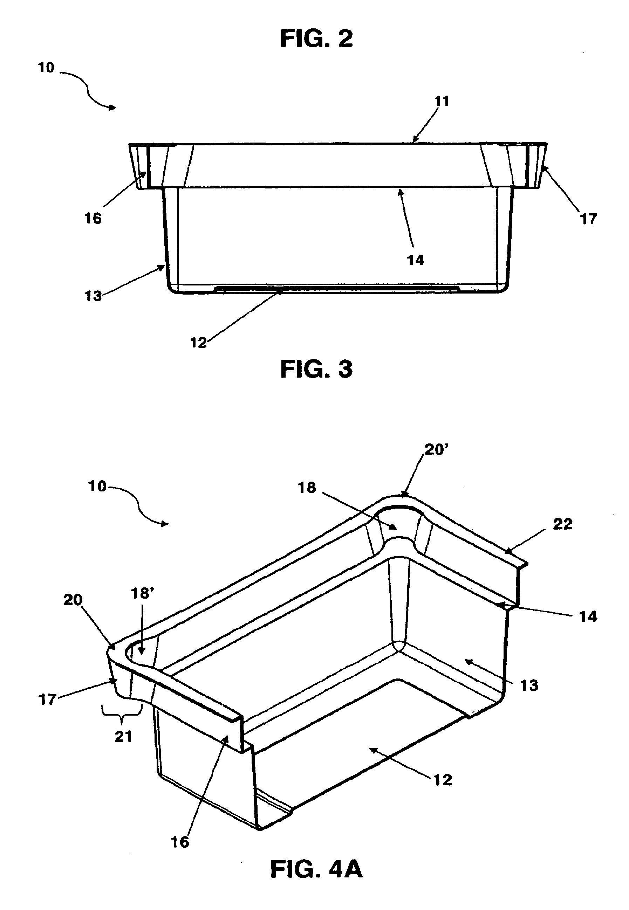

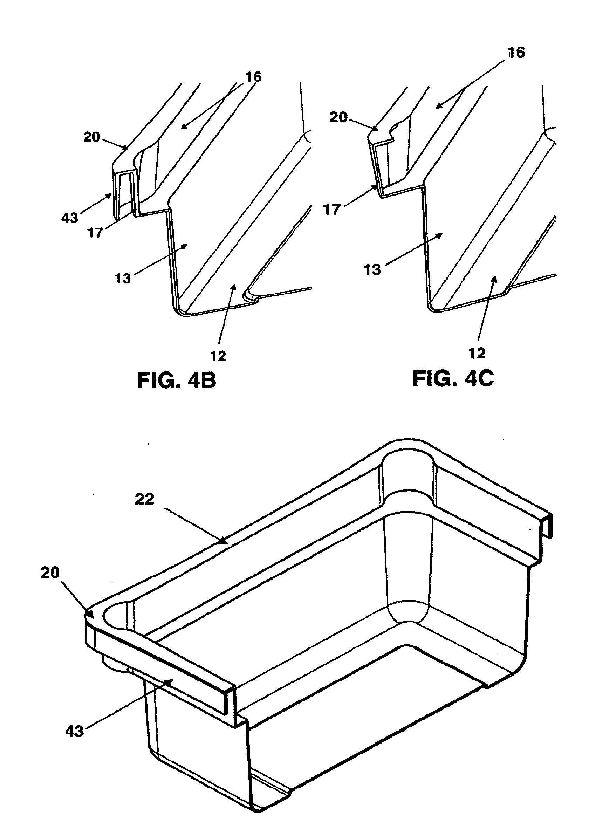

Selon ce premier aspect de l'invention, une boîte pour le stockage, le transport et la protection de contenants 28 suspendus en position verticale est fournie. La **Fig.** 2 représente une vue schématique du dessus de la boîte selon un second mode de réalisation particulier de l'invention. Ladite boîte 10 comprend un fond rectangulaire 12, des parois latérales inférieures 13 (non représentées sur cette vue) raccordées audit fond 12, et un redan 14 raccordé auxdites parois latérales inférieures et formant un appui pour supporter un plateau 15. Ladite boîte 10 comporte également des parois latérales supérieures 16 (non représentées sur cette vue) et un évidement 18 à chacun de ses coins supérieurs 21. Une bordure 20 est positionnée sur chacun des coins supérieurs de ladite boîte 10. Lesdites bordures 20 s'étendent vers l'intérieur de la boîte 10. Un rebord supérieur 22 est raccordé aux parois latérales supérieures 16, non représentées sur cette vue, et présente un bord périphérique 34 de forme concave. Le rebord supérieur 22 et ladite bordure 20 forment une surface continue susceptible d'être recouverte par un opercule. En référence à la Fig. 3 et aux Fig. 4a et 4c, lesdites parois latérales supérieures 16 comportent chacune au moins une et de préférence deux portions 17 s'étendant verticalement, obliquement ou de manière oblique inversée vers l'extérieur de la boîte 10, de sorte qu'une boîte 10 peut être disposée côte à côte avec une autre boîte du même type de manière à ce que les portions 17 de ces boîtes s'appuient les unes sur les autres laissant des espaces 41 entre eux, comme clairement montré à la **Fig. 5****.** Lesdites portions 17 s'étendent verticalement, de manière oblique ou de manière oblique inversée ce qui permet d'éviter le chevauchement, même lorsque deux boîtes voisines 10 ne sont pas exactement au même niveau. Les portions 17 s'étendant vers l'extérieur de la boîte évitent donc que les rebords supérieurs 22 n'entrent en contact entre eux et se chevauchent. Les boîtes connues de l'art antérieur, par exemple par la figure 6 de US 6,012,595**,** présentent au contraire des bords minces à une boîte adjacente de même type, ce qui peut occasionner des chevauchements si les bords minces de deux boîtes adjacentes ne coïncident pas à la même hauteur.

Ladite boîte 10 comprend donc une ouverture supérieure 11, un fond rectangulaire 12, des parois latérales inférieures 13 et un redan 14 formant un appui pour supporter un plateau. Il est en effet important de prémunir les contenants 28 de tout contact avec la boîte 10. Le plateau est utilisé pour séparer des seringues, contenues dans ledit plateau, du fond 12 de ladite boîte 10. Le fond rectangulaire 12 peut également contenir des gorges ou des rainures. Celles-ci peuvent servir à éviter le glissement entre deux surfaces de boîtes disposées l'une sur l'autre. En effet, si le dessus du plateau 15 d'une première boîte comprend des saillies, celles-ci pourront s'insérer dans lesdites rainures du fond 12.

En se référant encore à la **Fig.2** et **Fig.4A****,** ladite boîte 10 comprend également dans ses parois latérales supérieures 16, à ses quatre coins, des évidements 18, 18' pour permettre de saisir et retirer aisément, lorsque nécessaire, le plateau 15.

Ladite boîte comprend également une bordure 20 positionnée sur ses coins supérieurs 21, comme illustré à la **Fig. 2** et aux **Fig. 4A****,** **4B, 4C****.** Ladite bordure 20 et le rebord supérieur 22 forment une surface continue. Ladite surface continue est plane et peut donc favoriser et améliorer sa manipulation par un dispositif automatique.

De plus, lorsque ladite bordure 20 et le rebord supérieur 22 forment une surface plane, ceux-ci peut être surmontés par un opercule ou un couvercle pour favoriser l'empilage desdites boîtes les unes sur les autres et limiter les échanges entre l'intérieur et l'extérieur de ladite boîte 10.

En se référant à la **Fig. 3****,** ladite boîte 10 comprend un fond rectangulaire 12, des parois latérales inférieures 13 raccordées audit fond 12, un redan 14 raccordé auxdites parois latérales inférieures 13, une ouverture supérieure 11, des parois latérales supérieures 16 raccordées audit redan 14. Lesdites parois latérales supérieures comportent une portion 17 s'étendant de manière oblique inversée. Par ailleurs, en se référant à la **Fig. 5****,** le bord périphérique 34 dudit rebord supérieur 22 raccordé à une paroi latérale supérieure 16 est de forme concave. Ledit bord périphérique 34 ne s'étend pas au-delà du plan vertical formé par les portions 17 et 17'.

La présence des espaces 40 et 41 évite la superposition des boîtes 10, ce qui a pour conséquence de réduire sensiblement le risque de dommage ou de bris de contenants médicaux stockées même lorsque plusieurs boîtes sont disposés ensemble côte à côte et/ou les uns au-dessus des autres comme montré à la **Fig. 5** et à la **Fig. 6****.**

La **Fig. 4A** représente une vue en coupe de l'intérieur de la boîte selon le second mode de réalisation particulier de l'invention. Ladite boîte 10 comprend un fond 12 rectangulaire, des parois latérales inférieures 13 raccordées audit fond 12, un redan 14 raccordé auxdites parois latérales inférieures 13, des parois latérales supérieures 16 raccordées audit redan 14. Un rebord supérieur 22 est raccordé auxdites parois latérales supérieures 16. De plus ladite boîte comprend des évidements 18, 18' dans les coins supérieurs 21. Lesdits coins supérieurs 21 sont surmontés d'une bordure 20 formant une surface continue avec le rebord supérieur 22. Ladite bordure 20 s'étend vers l'intérieur de la boîte, comme représenté également à la **Fig. 4C****.** Les parois latérales supérieures 16 comportent chacune deux portions 17 s'étendant de manière oblique inversée et situées dans les coins supérieurs de ladite boîte 10. La manipulation de ladite boîte 10 par un dispositif automatisé est facilitée par la présence des évidements 18 et 18', des bordures 20 et 20'. La **Fig. 4B** représente une vue en coupe d'un coin de la boîte 10 selon un premier mode de réalisation particulier de l'invention. Ladite bordure 20 s'étend vers l'extérieur de la boîte 10 et comporte une paroi verticale 43, formant une jupe. Dans ce cas, le contact entre ceux boîtes peut s'effectuer par l'intermédiaire de ladite paroi verticale 43. Ladite paroi verticale 43 peut également se prolonger le long du bord supérieur 22. Ladite paroi verticale 43 peut également se prolonger sur toute la périphérie de la boîte comme représenté à la **Fig. 4D****.**

La **Fig. 9b** représente partiellement et en coupe une boîte 10 suivant un premier mode de réalisation de l'invention et une boîte 10' suivant un second mode de réalisation de l'invention. La jupe 43 de la boîte 10 s'étend verticalement en face de la portion 17 de la boîte 10' de sorte que, même en présence d'inégalités de hauteur, de chocs ou de vibration lors de la manipulation et du transport, une boîte ne pourra pas en chevaucher une autre. La **Fig. 9b** représente le cas où la jupe 43 s'étend verticalement et la portion 17 s'étend de manière oblique inversée, mais on comprendra aisément qu'une jupe 43 ou une portion 17 en position oblique aura le même effet. Des angles d'obliquité de 5° à 10° par rapport à la verticale dans l'un et l'autre sens, conviennent parfaitement. On comprendra également que l'effet d'anti-chevauchement est obtenu dès que deux boîtes présentent l'une à l'autre en saille une paroi de hauteur supérieure aux inégalités de hauteur et aux sauts provoqués lors du transport et de la manutention.

La **Fig. 6** est une vue en perspective montrant un rangement de plusieurs boîtes 10 du même type sur une palette 27 (format EuroPallet) côte à côte et les uns au-dessus des autres pour former un container 26 selon l'invention. Lesdites boîtes 10 peuvent également être contenues dans un élément de protection 28 tel qu'un sac ou un emballage 37. Ledit container 26 représenté à la **Fig. 6** peut optionnellement comprendre un moyen de contrôle de la qualité de ladite palette. Si lesdites boîtes sont contenues dans un sac ou un emballage 37, ledit moyen de contrôle de la qualité peut permettre de maintenir ledit objet à une pression inférieure à la pression atmosphérique. Ce type de rangement est avantageux pour le stockage, le transport au sein d'une installation industrielle, ou le transport (par route ou autre) entre deux installations industrielles distantes. Lesdites boîtes 10 peuvent contenir un plateau 15 susceptible d'être utilisé pour le rangement de contenants médicaux. Lesdites boîtes 10 peuvent également être surmontées d'un opercule ou d'un couvercle (non représenté sur la **Fig. 6****)** pour favoriser leur empilement et la stérilité vis-à-vis de l'environnement extérieur. Grâce à la structure spécifique de la boîte 10 selon l'invention, lesdites boîtes 10 présentes dans la palette ne se chevauchent pas. Le container 26 peut comporter des parois latérales verticales 35 et un couvercle rigide 36. Comme représenté à la **Fig. 6****,** le container peut également comprendre des sangles 39, une platine de localisation 38 et une housse 42. Lesdites sangles 39, ladite platine de localisation 38 et ladite housse 42 entourent ou supportent ladite pluralité de boîtes du container 26 et peuvent également servir de moyen de contrôle de la qualité dudit container 26.

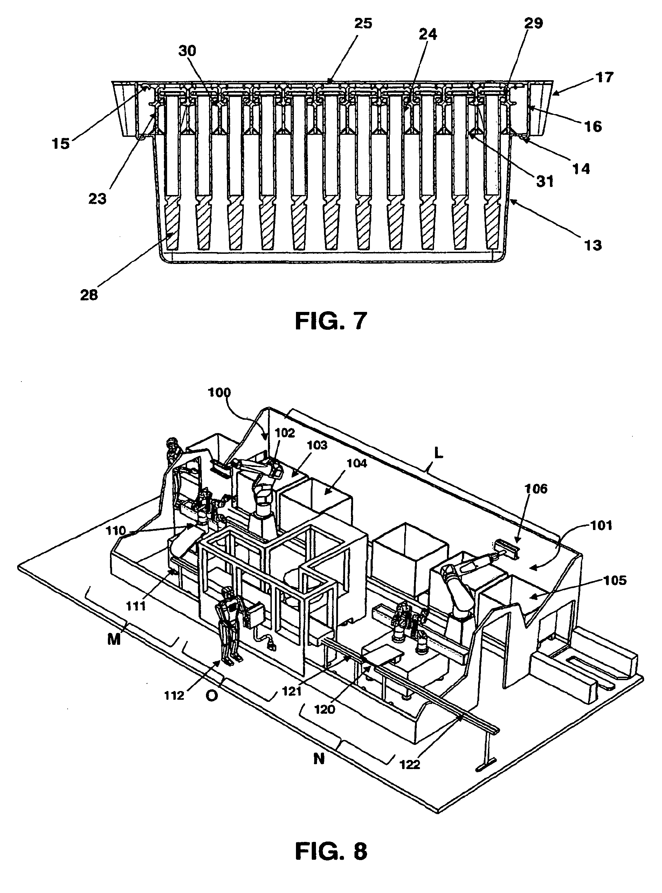

La **Fig.7** représente une vue schématique de la boîte selon l'invention comprenant un plateau. Ledit plateau 15 comprend une pluralité de rails 23 disposés parallèlement les uns aux autres sur une face dudit plateau. Des lèvres de support 30 sont agencées le long desdits rails 23, une lèvre 30 et un rail 23 forment ainsi avec une lèvre d'un rail adjacent une ouverture 24, une paire de lèvres adjacentes étant aptes à soutenir et stocker des contenants 28 par leurs flasques 29. Ledit plateau 15 comprend une paroi supérieure 25 perpendiculaire à ladite pluralité de rails 23. De plus, lesdits rails 23 et lesdites lèvres 30 sont en substance libre d'aspérités, permettant ainsi aux contenants 28 de glisser le long des rails 23. Ainsi lorsqu'un plateau 15 est inséré dans la boîte 10 de manière à former un conditionnement fermé, lorsqu'un tel conditionnement est disposé côte à côte avec un autre conditionnement du même type, les portions 17 respectives de chaque boîte s'appuient les unes contre les autres laissant un espace 40 entre les coins desdits boîtes 10.

Il faut remarquer que la boîte suivant l'invention est totalement compatible avec les plateaux de l'art antérieur (comme les plateaux-nests ou les plateaux peignes).

La **Fig. 8** représente une ligne de production utilisant des boîtes et des containers selon l'invention. Les boîtes et les containers selon l'invention peuvent facilement être manipulés par des dispositifs automatisés grâce à la configuration desdites boîtes empêchant leur chevauchement. La ligne de production peut donc être configurée, i.e. confiner dans un endroit clos, pour convenir à un environnement stérile nécessaire à la production de vaccins, par exemple soumis aux grades A, B, C ou D (norme GMP). De telles normes imposent des conditions de stérilité et de propreté très importantes et très coûteuses. Ainsi, limiter la présence d'opérateurs permet de diminuer les risques liés à la manipulation manuelle. De plus, les espaces de stockage et de travail sont nettement réduits, conduisant à une baisse des coûts de production. Ainsi, la ligne de production peut contenir plusieurs zones : une ou plusieurs zones destinées au convoyage des containers, une ou plusieurs zones de traitement des containers et des boîtes, une ou plusieurs zones d'activité sur les contenants contenus dans lesdites boîtes. Par exemple, comme représenté à la **Fig. 8****,** la ligne de production peut contenir une zone L de convoyage des containers, deux zones M et N de traitement des containers et des boîtes, et une zone O d'activité sur les contenants contenus dans lesdites boîtes. En l'absence de maintenance ou de travail spécifique sur la ligne de production à l'arrêt, cette dernière peut être isolée de l'environnement extérieur afin de préserver les conditions aseptiques nécessaires. La zone L peut comprendre un système de rails sur lesquels les containers selon l'invention sont convoyés. Cette zone peut contenir une ou plusieurs zones de chargement ou déchargement desdites boîtes 10 ou desdits containers 26 selon l'invention. Par exemple, ladite zone L peut contenir une première sous-zone 100 dans laquelle les containers 103 sont déchargés par un dispositif automatisé 102. Par exemple, lesdites boîtes peuvent contenir des seringues. Les boîtes (non représentées) contenues dans ledit container 103 sont acheminées par le dispositif 102 vers une première zone M de traitement desdites boîtes. La zone M peut contenir un dispositif de déchargement 110 des seringues rangées dans lesdites boîtes 10 qui sont ultérieurement placées sur un système de convoyage 111. Lesdites boîtes vides sont prises en charge par le système automatisé 102 et rangées dans un container 104. Le container 104 va être convoyé dans la zone L vers une seconde sous-zone 101. Les seringues déchargées dans la zone de traitement M sont amenées vers la zone O d'activité par l'intermédiaire du système de convoyage 111. L'activité peut être une inspection de la qualité des seringues pour évaluer la présence de défauts. Alternativement, l'activité peut concerner le remplissage des seringues avec une solution thérapeutiques comme un vaccin. Alternativement, l'activité peut également concerner l'étiquetage des seringues. Un opérateur 112 peut intervenir dans la programmation et la vérification du bon déroulement au niveau de la zone d'activité ou des zones de traitement. Une fois l'activité réalisée, les seringues sont convoyées vers une zone N de traitement par l'intermédiaire d'un système de convoyage approprié 121. Les seringues sont par exemple chargées dans lesdites boîtes (non représentées) à l'aide de système automatisé 120. Alternativement, les seringues peuvent être convoyées par l'intermédiaire d'un convoyeur by-pass 122 vers une autre ligne de production ou d'assemblage. La boîte 10 contenant les seringues est ensuite placée dans un container 105 situé dans la zone L sous-zone 101 à l'aide d'un dispositif automatisé adéquat 106. Les containers de la sous-zone 101 sont ensuite acheminés pour subir un traitement ultérieur ou pour être stocké. Grâce à l'utilisation de boîtes et de containers selon l'invention, la ligne de production peut être optimisée en termes d'efficacité, d'espace, de cadence et de qualité. L'absence de contact entre un opérateur et les contenants médicaux, par exemple les seringues ou des flacons, diminue donc les risques de contaminations ou de dommages desdits contenants médicaux. Les containers et les boîtes utilisés dans les zones L, M et N sont identiques et réutilisables dans la ligne de production.

## Revendications

1. Boîte (10), pour le stockage, le transport et la protection de contenants suspendus en position verticale, comprenant une ouverture supérieure (11), un fond rectangulaire (12), des parois latérales inférieures (13) se raccordant audit fond (12), un redan (14) se raccordant auxdites parois latérales inférieures (13) et formant un appui pour supporter un plateau, des parois latérales supérieures (16) se raccordant par leur extrémité inférieure audit redan (14) et par leur extrémité supérieure à un rebord supérieur (22) s'étendant vers l'extérieur de la boîte (10), une bordure (20) étant positionnée sur les coins supérieurs (21) de ladite boîte (10) et s'étendant vers l'intérieur de la boîte, ou s'étendant vers l'extérieur de la boîte et comportant une paroi verticale ; **caractérisée en ce que** lesdites parois latérales supérieures (16) de ladite boîte (10) comportent, au moins deux portions (17 ; 43) s'étendant vers l'extérieur de la boîte (10), de sorte que ladite boîte (10), lorsqu'elle est disposée côte à côte avec une seconde boîte selon l'invention (10), est en contact avec ladite seconde boîte par l'intermédiaire desdites portions (17, 43) empêchant ainsi le chevauchement de ladite boîte avec ladite seconde boîte; et **en ce que** ledit rebord supérieur (22) a un bord périphérique (34) situé en deçà du plan vertical formé par deux desdites portions (17 ; 43).

2. Boîte selon la revendication 1 **caractérisée en ce qu'**au moins une des portions (17 ; 43) est une jupe (43) raccordée au rebord supérieur (22) ou **en ce qu'**au moins une des portions (17) est raccordée au redan (14).

3. Boîte selon l'une quelconque des revendications précédentes, **caractérisée en ce qu'**au moins une des portions (17 ; 43) est située dans un coin de ladite boîte (10).

4. Boîte selon l'une quelconque des revendications précédentes, **caractérisée en ce qu'**elle comprend dans ses parois latérales supérieures (16) au moins un évidement (18) pour saisir et retirer aisément un plateau (15) de ladite boîte (10).

5. Boîte selon l'une quelconque des revendications précédentes, **caractérisée en ce que** ladite boîte (10) comporte des perforations (19) dans lesdites parois latérales supérieures (16) ou lesdites parois latérales inférieures (13) pour faciliter la stérilisation de ladite boîte (10) et de son contenu.

6. Boîte selon l'une quelconque des revendications précédentes, **caractérisée en ce que** ladite bordure (20) et ledit rebord supérieur (22) sont approximativement dans le même plan.

7. Boîte selon l'une quelconque des revendications précédentes, **caractérisée en ce que** ledit rebord supérieur (22) a un bord périphérique (34) de forme concave.

8. Boîte selon l'une quelconque des revendications précédentes, **caractérisée en ce qu'**elle comprend un plateau (15), ledit plateau (15) comportant une pluralité de rails (23) disposés parallèlement les uns aux autres sur une face dudit plateau, des lèvres de support (30) étant agencées le long desdits rails (23), une lèvre (30) et un rail (23) formant ainsi avec une lèvre d'un rail adjacent une ouverture (24), une paire de lèvres adjacentes étant aptes à soutenir et stocker des contenants (28) par leurs flasques (29), et **en ce que** ledit plateau (15) comprend une paroi supérieure (25) perpendiculaire à ladite pluralité de rails (23) et que lesdits rails et lesdites lèvres sont en substance libre d'aspérités, permettant ainsi aux contenants (28) de glisser le long des rails (23).

9. Container pour le transport de boîtes **caractérisé en ce qu'**il comprend une palette (27) sur laquelle est disposée une pluralité de boîtes (10) selon l'une quelconque des revendications 1 à 8, et comprenant optionnellement un moyen de contrôle de la qualité dudit container.

10. Container selon la revendication précédente **caractérisé en ce qu'**il comprend également des parois latérales verticales (35) et un couvercle rigide (36).

11. Méthode de palettisation automatique **caractérisée en ce qu'**elle comprend les étapes suivantes de :
a) mettre à disposition une pluralité de boîtes (10) selon l'une quelconque des revendications 1 à 8, et une palette (27),
b) disposer à l'aide d'un dispositif automatisé sur ladite palette (27), ladite pluralité de boîtes côte à côte ou l'une sur l'autre, tel que les portions (17) desdites parois latérales supérieures (16) desdites boîtes (10) selon l'invention s'appuient les unes contre les autres, les empêchant de se chevaucher,
c) fournir un container (26) selon l'une des revendications 9 ou 10.

12. Méthode de dé-palettisation automatique **caractérisée en ce qu'**elle comprend les étapes suivantes de :
a) mettre à disposition un container (26) selon l'une des revendications 9 ou 10, comprenant une palette (27) sur laquelle est disposée une pluralité de boîtes (10) selon l'une quelconque des revendications 1 à 8,
b) retirer ladite pluralité de boîtes de ladite palette par l'intermédiaire d'un système automatisé manipulant lesdites boîtes par l'intermédiaire de leurs parois latérales inférieures (13), leurs parois latérales supérieures (16), leurs évidements (18) ou par la bordure (20) positionnée au dessus des coins supérieurs (21) de ladite boîte (10).

13. Utilisation de la boîte selon l'une quelconque des revendications 1 à 8 pour le stockage, le transport et la protection de contenants suspendus dans une position verticale.

## Patentansprüche

1. Box (10) zum Lagern, zum Transport und zum Schutz von in senkrechter Stellung aufgehängten Behältern, die eine obere Öffnung (11), einen rechtwinkligen Boden (12), untere Seitenwände (13), die mit dem Boden (12) verbunden sind, eine Stufe (14), die mit den unteren Seitenwänden (13) verbunden ist und eine Auflage zum Tragen einer Platte bildet, obere Seitenwände (16), die über ihr unteres Ende mit der Stufe (14) und über ihr oberes Ende mit einer oberen Randleiste (22) verbunden sind, die sich nach außerhalb der Box (10) erstreckt, und eine Umrandung (20) aufweist, die auf den oberen Ecken (21) der Box (10) positioniert ist und sich zur Innenseite der Box erstreckt, oder sich zur Außenseite der Box erstreckt und eine senkrechte Wand aufweist, **dadurch gekennzeichnet, dass** die oberen Seitenwände (16) der Box (10) mindestens zwei Abschnitte (17; 43) aufweisen, die sich zur Außenseite der Box (10) erstrecken, so dass die Box (10), wenn sie neben einer zweiten erfindungsgemäßen Box (10) angeordnet wird, mit der zweiten Box über die Abschnitte (17, 43) in Kontakt ist, wodurch das Überlappen der Box mit der zweiten Box verhindert wird; und dass die obere Randleiste (22) einen Umfangsrand (34) hat, der sich diesseits der von zwei der Abschnitte (17; 43) gebildeten senkrechten Ebene befindet.

2. Box nach Anspruch 1, **dadurch gekennzeichnet, dass** mindestens einer der Abschnitte (17; 43) eine Schürze (43) ist, die mit der oberen Randleiste (22) verbunden ist, oder dass mindestens einer der Abschnitte (17) mit der Stufe (14) verbunden ist.

3. Box nach einem der vorhergehenden Ansprüche, **dadurch gekennzeichnet, dass** mindestens einer der Abschnitte (17; 43) sich in einer Ecke der Box (10) befindet.

4. Box nach einem der vorhergehenden Ansprüche, **dadurch gekennzeichnet, dass** sie in ihren oberen Seitenwänden (16) mindestens eine Aussparung (18) enthält, um eine Platte (15) bequem zu ergreifen und aus der Box (10) zu entnehmen.

5. Box nach einem der vorhergehenden Ansprüche, **dadurch gekennzeichnet, dass** die Box (10) Perforationen (19) in den oberen Seitenwänden (16) oder den unteren Seitenwänden (13) aufweist, um die Sterilisierung der Box (10) und ihres Inhalts zu vereinfachen.

6. Box nach einem der vorhergehenden Ansprüche, **dadurch gekennzeichnet, dass** die Umrandung (20) und die obere Randleiste (22) sich in etwa in der gleichen Ebene befinden.

7. Box nach einem der vorhergehenden Ansprüche, **dadurch gekennzeichnet, dass** die obere Randleiste (22) einen Umfangsrand (34) konkaver Form hat.

8. Box nach einem der vorhergehenden Ansprüche, **dadurch gekennzeichnet, dass** sie eine Platte (15) enthält, wobei die Platte (15) eine Vielzahl von Schienen (23) aufweist, die parallel zueinander auf einer Seite der Platte angeordnet sind, wobei Trägerlippen (30) entlang der Schienen (23) angeordnet sind, wobei eine Lippe (30) und eine Schiene (23) so mit einer Lippe einer benachbarten Schiene eine Öffnung (24) bilden, wobei ein Paar von benachbarten Lippen Behälter (28) über ihre Flansche (29) tragen und lagern können, und dass die Platte (15) eine obere Wand (25) lotrecht zur Vielzahl von Schienen (23) enthält, und dass die Schienen und die Lippen im Wesentlichen frei von Rauigkeiten sind, was es den Behältern (28) ermöglicht, entlang der Schienen (23) zu gleiten.

9. Container für den Transport von Boxen, **dadurch gekennzeichnet, dass** er eine Palette (27) enthält, auf der eine Vielzahl von Boxen (10) nach einem der Ansprüche 1 bis 8 angeordnet ist, und optional eine Einrichtung zur Überprüfung der Qualität des Containers enthält.

10. Container nach dem vorhergehenden Anspruch, **dadurch gekennzeichnet, dass** er ebenfalls senkrechte Seitenwände (35) und einen steifen Deckel (36) enthält.

11. Automatisches Palettisierungsverfahren, **dadurch gekennzeichnet, dass** es die folgenden Schritte enthält:
a) Bereitstellen einer Vielzahl von Boxen (10) nach einem der Ansprüche 1 bis 8 und einer Palette (27),
b) Anordnen der Vielzahl von Boxen nebeneinander oder übereinander auf der Palette (27) mit Hilfe einer automatisierten Vorrichtung, derart, dass die Abschnitte (17) der oberen Seitenwände (16) der erfindungsgemäßen Boxen (10) sich gegeneinander anlehnen, was sie daran hindert, sich zu überlappen,
c) Liefern eines Containers (26) nach einem der Ansprüche 9 oder 10.

12. Automatisches Entpalettisierungsverfahren, **dadurch gekennzeichnet, dass** es die folgenden Schritte enthält:
a) Bereitstellen eines Containers (26) nach einem der Ansprüche 9 oder 10, der eine Palette (27) enthält, auf der eine Vielzahl von Boxen (10) nach einem der Ansprüche 1 bis 8 angeordnet ist,
b) Entfernen der Vielzahl von Boxen von der Palette mittels eines automatisierten Systems, das die Boxen mittels ihrer unteren Seitenwände (13), ihrer oberen Seitenwände (16), ihrer Aussparungen (18) oder der Umrandung (20) manipuliert, die über den oberen Ecken (21) der Box (10) positioniert ist.

13. Verwendung der Box nach einem der Ansprüche 1 bis 8 zum Lagern, zum Transport und zum Schutz von Behältern, die in einer senkrechten Stellung aufgehängt sind.

## Claims

1. Box (10), for storing, transporting and protecting vertically-suspended containers, comprising a top opening (11), a rectangular bottom (12), lower side walls (13) connecting to said bottom (12), a step (14) connecting to said lower side walls (13) and forming a bearing supporting a plate, upper side walls (16) connecting by their bottom end to said step (14) and by their top end to a top flange (22) extending toward the outside of the box (10), a rim (20) being positioned on the top corners (21) of said box (10) and extending toward the interior of the box, or extending toward the outside of the box and including a vertical wall; **characterized in that** said upper side walls (16) of said box (10) include at least two portions (17; 43) extending toward the outside of the box (10), so that said box (10), when it is positioned side-by-side with a second box (10) according to the invention, is in contact with said second box via said portions (17, 43) thus preventing the overlapping of said box with said second box; and **in that** said top flange (22) has a peripheral edge (34) situated short of the vertical plane formed by two of said portions (17; 43).

2. Box according to Claim 1, **characterized in that** at least one of the portions (17; 43) is a skirt (43) connected to the top flange (22) or **in that** at least one of the portions (17) is connected to the step (14).

3. Box according to either of the preceding claims, **characterized in that** at least one of the portions (17; 43) is situated in a corner of said box (10).

4. Box according to any one of the preceding claims, **characterized in that** it comprises, in its upper side walls (16) at least one void (18) for easily grasping and removing a plate (15) from said box (10).

5. Box according to any one of the preceding claims, **characterized in that** said box (10) includes perforations (19) in said upper side walls (16) or said lower side walls (13) to facilitate the sterilization of said box (10) and its content.

6. Box according to any one of the preceding claims, **characterized in that** said rim (20) and said top flange (22) are approximately in the same plane.

7. Box according to any one of the preceding claims, **characterized in that** said top flange (22) has a concave peripheral edge (34).

8. Box according to any one of the preceding claims, **characterized in that** it comprises a plate (15), said plate (15) including a plurality of rails (23) positioned parallel to one another on one face of said plate, support lips (30) being arranged along said rails (23), a lip (30) and a rail (23) thus forming, with a lip of an adjacent rail, an opening (24), a pair of adjacent lips being suitable for supporting and storing containers (28) by their flanges (29), and **in that** said plate (15) includes an upper wall (25) perpendicular to said plurality of rails (23) and that said rails and said lips are substantially free of irregularities, thus enabling the containers (28) to slide along the rails (23).

9. Container for transporting boxes, **characterized in that** it comprises a pallet (27) on which is arranged a plurality of boxes (10) according to any one of Claims 1 to 8, and optionally including a means for checking the quality of said container.

10. Container according to the preceding claim, **characterized in that** said container also has vertical side walls (35) and a rigid lid (36).

11. Automatic palletization method, **characterized in that** it comprises the following steps of:
a) providing a plurality of boxes (10) according to any one of Claims 1 to 8, and a pallet (27),
b) using an automated device, arranging on said pallet (27) said plurality of boxes side by side or one on top of the other, such that the portions (17) of said upper side walls (16) of said boxes (10) according to the invention bear against one another, preventing them from overlapping,
c) supplying a container (26) according to either of Claims 9 and 10.

12. Automatic depalletization method, **characterized in that** it comprises the following steps of:
a) providing a container (26) according to either of Claims 9 and 10, comprising a pallet (27) on which is arranged a plurality of boxes (10) according to any one of Claims 1 to 8,
b) removing said plurality of boxes from said pallet via an automated system handling said boxes via their lower side walls (13), their upper side walls (16), their voids (18) or by the rim (20) positioned above the top corners (21) of said box (10).

13. Use of the box according to any one of Claims 1 to 8 for storing, transporting and protecting vertically-suspended containers.
